# EUROPEAN PATENT SPECIFICATION

(11) **EP 0 960 620 B1**
(45) Date of publication and mention of the grant of the patent: **11.01.2006**
(21) Application number: 98123251.5
(22) Date of filing: 07.12.1998
(51) Int. Cl.: A61K 31/44, A61K 47/32, A61K 9/48

(54) **A stable oral pharmaceutical composition containing a substituted pyridylsulfinyl benzimidazole**
Stabile orale pharmazeutische Zusammensetzung enthaltend ein Pyridylsulfinyl-substituiertes Benzimidazole
Composition pharmaceutique orale stable contenant un pyridylsulfinyl-benzimidazole substitué

(30) Priority: 28.05.1998 US 86224
(43) Date of publication of application: 01.12.1999
(73) Proprietor: Ranbaxy Laboratories Limited, New Delhi 110 019 (IN)
(72) Inventor: Thacharodi, Dilip Kumar, Gurgaon-122001, Haryana (IN); Kampal, Ashok, Amritsar-143001, Punjals (IN)
(74) Representative: Gilholm, Stephen Philip

(56) References cited:
- EP-A- 0 519 365
- WO-A-96/01623
- WO-A-97/12580
- US-A- 5 178 867

## Description

### Background of the Invention

The present invention relates to a stable oral pharmaceutical composition comprising a substituted pyridylsulfinyl benzimidazole having gastric acid secretion inhibitory activity as the active ingredient and a carrier which acts as a stabilizing excipient. The invention also relates to a process for making the pharmaceutical composition.

United States Patent Nos. 4,255,431; 4,628,098; and 4,758,579 disclose substituted pyridylsulfinyl benzimidazoles (such as omeprazole) as potent inhibitors of gastric acid secretion. This class of compounds inhibits gastric acid secretion by inhibiting H⁺-K⁺ ATPase (proton pump) activity. Drugs in this class are known to be highly unstable in an acidic environment. They are also unstable in the presence of moisture and organic solvents. Thus, the formulation in which the drugs are to be administered to a patient, and the process for manufacture of the formulation, must be designed to protect the drug from moisture as well as an acidic environment. Due to the very rapid drug degradation which occurs in acidic gastric fluids, the formulations should also be enteric coated.

United States Patent No. 4,786,505 discloses an oral pharmaceutical composition comprising a core containing omeprazole together with an alkaline reacting compound, or an alkaline salt of omeprazole optionally together with an alkaline compound; one or more subcoating layers comprising inert reacting compounds which are soluble or rapidly disintegrating in water, or polymeric, water soluble film forming compounds, optionally containing pH-buffering alkaline compounds; and an outer enteric coat. The alkaline reacting compound is a pharmaceutically acceptable substance (or substances) which creates a "micro-pH" around each omeprazole particle of not less than pH=7, preferably not less than pH=8, when water is adsorbed onto the particles of the mixture or when water is added in small amounts to the mixture. The subcoating layer separates the omeprazole containing core from the enteric coating polymer(s) containing free carboxyl groups. The enteric coating polymers can otherwise cause degradation of omeprazole during the coating process or during storage.

Japanese Patent 05-194,225 discloses tablets, granules and capsule formulations where the benzimidazole gastric ulcer inhibitors are stabilized by compounding with amino acids and buffering agents.

United States Patent No. 5,385,739 discloses a stable microgranule formulation containing a neutral core of sugar and starch and an active layer consisting of a dilution of omeprazole in mannitol in substantially equal amounts, wherein the active omeprazole layer contains about 10% by weight of carboxymethylstarch, and about 5% by weight of sodium lauryl sulfate, and wherein the dilution of omeprazole in mannitol is applied to the neutral core by means of hydroxypropyl methylcellulose as a high viscosity binder.

PCT Int. Pat. Appl. WO 97/12581 discloses a composition comprising: (a) a core containing omeprazole as the active principle, the core being constituted of nuclei and the omeprazole active principle mixed together and then compressed together, the omeprazole active principle not being in the form of an alkaline salt; (b) an intermediate layer; and (c) an enteric layer. The composition disclosed therein is stated to be free of alkaline reacting compounds which had previously been considered as essential; however, each of the compositions exemplified in WO 97/12581 contains either a lubricant, such as sodium stearyl fumarate, magnesium stearate, or talc in the core, or talc in the intermediate layer. These compounds are alkali metal or alkaline earth metal salts and are known to be alkaline in nature.

WO 96/01623 discloses an oral pharmaceutical multiple unit tableted dosage form comprising tablet excipients and individually enteric coated layered units of a core material containing active substances such as omeprazole optionally mixed with alkaline compounds covered with one or more layers of which at least one is an enteric coating layer. The application does not disclose a use of polyvinylpyrrolidone as a stabilizing agent for omeprazole. Example 8 of the application discloses a composition containing omeprazole and polyvinylpyrrolidone with the omeprazole to polyvinylpyrrolidone ratio being 6:1.

U.S. Patent No. 5,178,867 describes a drug delivery system comprised of a compartment, a semi-permeable wall, means in the wall for increasing the fluid passage into the dosage form, and a passageway in the wall for delivering the drug from the dosage form. The use of polyvinylpyrrolidone of a fluid flux agent for increasing the volume of fluid passage in the compartment is described. Additionally, as the claims of the present application are limited to crosslinked polyvinylpyrrolidone which is water-insoluble, such polyvinylpyrrolidone would not be usable as a fluid flux agent.

European Patent Application No. 0 519 365 describes an oral presentation form of pantoprazole, which consist of a core, an intermediate layer and an outer layer which is resistant to gastric juice.

International Patent application No. WO 97/12580 describes an oral composition comprising a core containing an acid labile benzimidazole, e.g. pantoprazole, an intermediate layer and an enteric layer. Omeprazole is specifically disclaimed in the claims of the application.

It is an object of the present invention to provide a stable oral pharmaceutical composition containing a substituted pyridylsulfinyl benzimidazole having gastric acid secretion inhibitory activity as the active ingredient and a carrier, which composition is free of alkaline compounds.

It is a further object of the present invention to provide a process for the preparation of a stable oral pharmaceutical composition in the form of a mixture containing a substituted pyridylsulfinyl benzimidazole and a carrier, wherein the mixture is not compressed to form hard intact core units, but is filled in the form of a powder or granules into enteric-coated capsules or capsules made from an enteric material. Once the capsules dissolve, the drug particles are freely dispersed in the gastrointestinal fluid so as to result in a rapid rate of dissolution and absorption.

It is a further object of the present invention to provide a process for the preparation of a stable oral pharmaceutical composition in the form of a mixture containing substituted pyridylsulfinyl benzimidazole which process is simple, less time consuming, and more economical than prior art processes.

### Summary of the Invention

It has been surprisingly found that in a mixture comprising a substituted pyridylsulfinyl benzimidazole and one or more polymers obtained by the polymerization of monomers at least one of which is vinylpyrrolidone, the substituted pyridylsulfinyl benzimidazole is stabilized. The mixture, even though it is free of alkaline reacting compounds, does not show a change in color which is typically observed in compositions where the benzimidazole has undergone degradation.

Accordingly, the present invention provides a pharmaceutical composition which is stable and suitable for oral administration to a patient, comprising a mixture of a substituted pyridylsulfinyl benzimidazole having gastric acid secretion inhibitory activity in an amount sufficient to inhibit gastric acid secretion in said patient, and a pharmaceutically acceptable carrier, said carrier comprising at least one polymer which is at least partially comprised of vinylpyrrolidone monomeric units. Optionally, the mixture also contains other pharmaceutically acceptable excipients. Desirably, the composition is in the form of a simple powder blend or granules of the active ingredient and the carrier, together with any optionally included excipients, filled into an enteric capsule, i.e., a capsule which is coated with an enteric polymer or which is made from an enteric polymer.

The present invention also provides a process for making a pharmaceutical composition which is stable and suitable for oral administration to a patient, comprising mixing together a substituted pyridylsulfinyl benzimidazole having gastric acid secretion inhibitory activity with a pharmaceutically acceptable carrier, the carrier comprising at least one polymer which is at least partially comprised of vinylpyrrolidone monomeric units, together with any optionally included pharmaceutically acceptable excipients. The mixture, which is in the form of a simple powder blend is then filled into enteric capsules, i.e., capsules which are coated with an enteric polymer or which are made from an enteric polymer. Alternatively, the mixture, in the form of a powder blend, is converted into granules and the granules are filled into an enteric capsule.

Whereas the processes for preparation of the prior art compositions involve steps for conversion of a powder blend into core units such as granules, pellets or tablets, and also the step of applying a subcoat over the core units, the process for preparation of the pharmaceutical composition according to the present invention does not require these steps because the powder blend or granules are filled into enteric capsules without being compressed into intact core units, and without applying a subcoat. Furthermore, whereas the core units of prior art compositions contain several pharmaceutical excipients, the pharmaceutical composition of the present invention may be a simple mixture comprising a substituted pyridylsulfinyl benzimidazole and one or more polymers obtained by the polymerization of monomers at least one of which is vinylpyrrolidone. For the above cited reasons, the process for the preparation of the present invention is simple, less time consuming and more economical than prior art processes.

### Detailed Description of the Invention

In accordance with the present invention, the substituted pyridylsulfinyl benzimidazole having gastric acid secretion inhibitory activity may be one of the following:
(a) a substituted pyridylsulfinyl benzimidazole having the structure of Formula I shown below 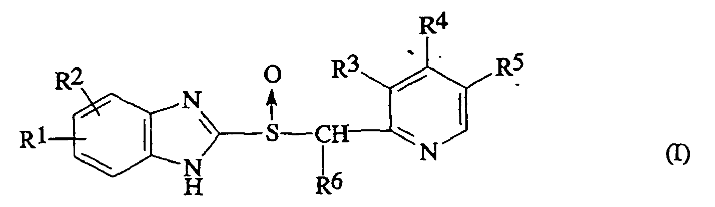
   wherein R¹ and R² are the same or different, and each of R¹ and R² is selected from the group consisting of hydrogen, halogen, carbomethoxy, carboethoxy, and C₁₋₄ alkyl, alkoxy, or alkanoyl in any position; R⁶ is selected from the group consisting of hydrogen, methyl, and ethyl; R³ and R⁵ are the same or different, and each is selected from the group consisting of hydrogen, methyl, methoxy, ethoxy, methoxyethoxy, and ethoxyethoxy; and R⁴ is methoxy, ethoxy, methoxyethoxy, or ethoxyethoxy; wherein R³, R⁴ and R⁵ are not all hydrogen; OR
(b) a substituted pyridylsulfinyl benzimidazole having the structure of Formula II shown below 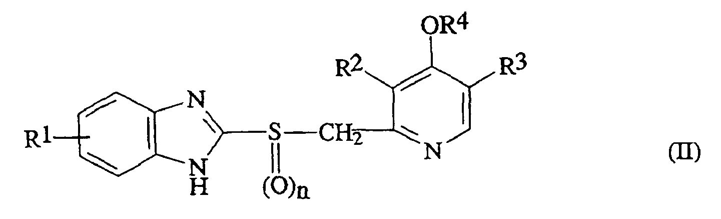
   wherein R¹ is hydrogen, methoxy or trifluoromethyl; R² and R³ are independently hydrogen or methyl; R⁴ is a C₂₋₅ fluorinated alkyl; and n is 0 or 1; OR
(c) a substituted pyridylsulfinyl benzimidazole having the structure of Formula III shown below 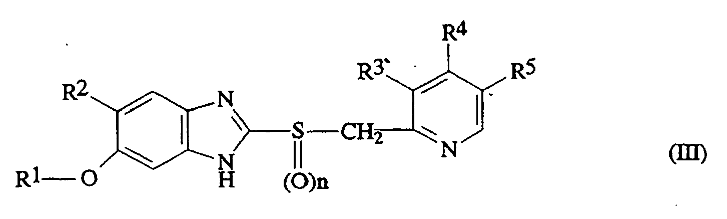
   wherein R¹ is C₁₋₃ alkyl which is at least partially substituted by fluorine, or chlorodifluoromethyl; R² is hydrogen, halogen, trifluoromethyl, C₁₋₃ alkyl, or C₁₋₃ alkoxy which may be partially or completely substituted by fluorine; or R¹ and R² together with the oxygen atom to which R¹ is bonded is C₁₋₂ alkylenedioxy which may be partially or completely substituted by fluorine, or chlorotrifluoroethylenedioxy; R⁴ is C₁₋₃ alkoxy; one of R³ and R⁵ is C₁₋₃ alkoxy and the other is hydrogen or C₁₋₃ alkyl; and n is 0 or 1.

Examples of substituted pyridylsulfinyl benzimidazoles that may be used as the active ingredient in the novel compositions of the present invention include omeprazole falling within the definition of Formula I, lansoprazole falling within the definition of Formula II, and pantoprazole falling within the definition of Formula III. The amount of the active agent used in the composition is that which will deliver a suitable therapeutically effective dose, i.e., an amount sufficient to inhibit gastric acid secretion in a patient, on a suitable daily dosing regimen.

According to the present invention, in addition to the substituted pyridylsulfinyl benzimidazole, the pharmaceutical composition contains a carrier comprising one or more polymers that are obtained by polymerization of monomers at least one of which is vinylpyrrolidone.

An example of a class of polymers that may be used in the present invention is polyvinylpyrrolidones also known as povidone or PVP. The United States Pharmacopoeia XXII describes povidone as a synthetic polymer consisting essentially of linear 1-vinyl-2-pyrrolidone groups. The polyvinylpyrrolidones are commonly available from BASF under the brand name Kollidon or from ISP under the brand name Plasdone. Polyvinylpyrrolidone is available as a water soluble polymer or as a cross-linked water insoluble polymer. Examples of water soluble polyvinylpyrrolidones include PVP K-12, PVP K-15, PVP K-17, PVP K-25, PVP K-30, PVP K-60, PVP K-90, and PVP K-120 having approximate molecular weights of 2500, 8000, 10000, 30000, 50000, 400000, 1000000, and 3000000, respectively. Soluble PVP is conventionally used as a binder in tablet formulations. In the present invention, soluble PVP is used in the inventive composition as a stabilizing excipient and as a diluent for the substituted pyridylsulfinyl benzimidazole.

Cross-linked polyvinylpyrrolidone is a polymer obtained by a polymerization process that produces a physically cross-linked polyvinylpyrrolidone (United States Patent No. 3,933,766) which is insoluble in water and in all the usual solvents. Examples of cross-linked polyvinylpyrrolidones that may be used in the present invention include various grades such as those available from BASF under the brand names Kollidon CL, Crospovidone M, and Kollidon CL-M. Because of its high swelling ability, cross-linked polyvinylpyrrolidone is conventionally used as a disintegrant in tablets; however, in the present invention it is used as a stabilizing excipient and as a diluent for the substituted pyridylsulfinyl benzimidazole.

Another example of a class of polymers that may be used in the present invention are water soluble vinylpyrrolidone-vinyl acetate copolymers that are formed by the copolymerization of vinylpyrrolidone and vinyl acetate. An example of a vinylpyrrolidone-vinyl acetate copolymer that may be used in the present invention is the copolymer available from BASF under the brand name Kollidon VA-64. In the present invention, the vinylpyrrolidone-vinyl acetate copolymer is used as a stabilizing excipient and as a diluent for the substituted pyridylsulfinyl benzimidazole.

According to the present invention, the pharmaceutically acceptable carrier is present in an amount from about 10% to about 98%, preferably from about 50% to about 90%, by weight of the total weight of the composition.

According to the present invention, the pharmaceutical composition may also contain conventional pharmaceutically acceptable excipients. Pharmaceutical excipients well known in the pharmaceutical arts can be found listed in the Handbook of Pharmaceutical Excipients (Ed. A. Wade and P.J. Weller, The Pharmaceutical Press, London), in the U.S. FDA listing of inactive ingredients, and in other sources of pharmaceutical literature.

In preferred embodiments, the pharmaceutically acceptable excipients may comprise fatty acid glycerides. One example of fatty acid glycerides that may be used in the invention is a mixture of glycerides (e.g., mono-, di- and/or triglycerides) of long chain (e.g., C₁₂ - C₁₈) fatty acids; for example, the range of products available under the brand name Gelucire (Gattefosse Corporation). Another example of fatty acid glycerides that may be used in the invention is a mixture of glycerides (e.g., triglycerides) of medium chain length (e.g., C₈ - C₁₀) fatty acids; for example, the range of products available under the brand names Miglyol, Crodamol GTC/C, MCT oil, Neobee M5, AKOMED, Nesatol, and the like. The fatty acid glycerides included in the composition of this invention can also be in the form of vegetable oils, such as castor oil, hydrogenated castor oil, or hydrogenated vegetable glycerides, such as those available under the brand name Witepsol.

According to the process of the present invention, a substituted pyridylsulfinyl benzimidazole having gastric acid secretion inhibitory activity, a carrier comprising one or more polymers comprising vinylpyrrolidone monomeric units, optionally together with pharmaceutically acceptable excipients, are mixed together to obtain a blend or granules, the blend or granules so obtained are filled into capsules, and the capsules are then enteric coated, or the blend or granules are filled into capsules having an enteric coating or made from an enteric material. In embodiments where one of the pharmaceutically acceptable excipients is a fatty acid glyceride, a liquid fatty acid glyceride is mixed with the other ingredients of the composition, or a solid fatty acid glyceride is first heated to above its melting point and the liquid obtained mixed with other ingredients of the composition to obtain granules.

The capsules used in the invention may be hard or soft capsules. The outer shell of the capsules may be composed of a film forming agent or agents, water and plasticizer. The shell may also contain coloring and opacifying agents. Examples of film forming agents which may be used in the capsule shell include gelatin, methylcellulose, hydroxypropyl methylcellulose, hydroxypropyl cellulose, and the like. When the shell is of a conventional type, e.g., it is made from gelatin, it is given an outer enteric coat. Alternatively, the capsules are enteric capsules wherein the shell itself is enteric in nature. The shell of enteric capsules may be made from one or more film forming polymers at least one of which has an enteric nature. The composition of enteric capsules is a known art. For example, the shell may be made from a mixture of polymers such as gelatin or hydroxypropyl methylcellulose, and one or more enteric polymers, such as a polyacrylate enteric polymer, cellulose acetate phthalate, hydroxypropyl methyl cellulose acetate succinate, or cellulose acetate butyrate; or from a mixture of gelatin or hydroxypropyl methylcellulose and polyvinyl acetate phthalate; or from calcium alginate and the like. The enteric polymers may be present as free acids or their salts. When the shell is of a conventional type, an outer enteric coating may be applied using known art. The coating composition may be aqueous or organic solvent based. The drying of the applied layers of a coating composition may be achieved by conventional means or by application of vacuum.

In another embodiment of the process of the present invention, a substituted pyridylsulfinyl benzimidazole having gastric acid secretion inhibitory activity, a carrier comprising one or more vinylpyrrolidone polymers, optionally together with pharmaceutically acceptable excipients, are mixed together to obtain a powder blend, and the blend so obtained is subjected to conventional processing steps to obtain granules or tablets.

The present invention is further illustrated by the following examples.

### EXAMPLE 1

Omeprazole and cross-linked polyvinylpyrrolidone in amounts as given in Table 1 were mixed together. The blend so obtained was filled into capsules.

**TABLE 1**

| **Ingredient** | **Weight (mg/capsule)** |
|---|---|
| Omeprazole | 20.00 |
| Cross-linked polyvinylpyrrolidone (Kollidon CL-M) - | 100.00 |
| Total | 120.00 |

The capsules were enteric coated in a Freund Hi-coater to a weight build-up of 10% using the coating composition as given in Table 2.

**TABLE 2**

| **Ingredient** | **Weight (g)** |
|---|---|
| Eudragit L - 100 - 55 | 100.00 |
| Sodium hydroxide | 1.40 |
| Titanium dioxide | 1 .70 |
| Talc | 50.00 |
| Polyethylene glycol-300 | 20.00 |
| Water | 650.00 |

### EXAMPLE 2

Omeprazole and vinylpyrrolidone-vinyl acetate copolymer in amounts as given in Table 3 were mixed together. The blend so obtained was filled into capsules.

**TABLE 3**

| **Ingredient** | **Weight (mg/capsule)** |
|---|---|
| Omeprazole | 20.00 |
| Vinylpyrrolidone-vinyl acetate copolymer (Kollidon VA-64) | 100.00 |
| Total | 120.00 |

The capsules were enteric coated in a Freund Hi-coater to a weight build-up of 10% using the coating composition as given in Table 2.

### EXAMPLE 3

Omeprazole, vinylpyrrolidone-vinyl acetate copolymer, and cross-linked polyvinylpyrrolidone in amounts as given in Table 4 were mixed together. The blend was filled into capsules.

**TABLE 4**

| **Ingredient** | **Weight (mg/capsule)** |
|---|---|
| Omeprazole | 20.00 |
| Cross-linked polyvinylpyrrolidone (Kollidon CL-M) | 50.00 |
| Vinylpyrrolidone-vinyl acetate copolymer (Kollidon VA-64) | 50.00 |
| Total | 120.00 |

The capsules were enteric coated in a Freund Hi-coater to a weight build-up of 10% using the coating composition as given in Table 2.

### EXAMPLE 4

Omeprazole and polyvinylpyrrolidone (PVP K 30) in the amounts as given in Table 5 were mixed together. The blend so obtained was filled into capsules.

**TABLE 5**

| **Ingredient** | **Weight (mg/capsule)** |
|---|---|
| Omeprazole | 20.00 |
| PVP K30 | 100.00 |
| Total | 120.00 |

The capsules were enteric coated in a Freund Hi-coater to a weight build-up of 10% using the coating composition as given in Table 2.

### EXAMPLE 5

Omeprazole and the other ingredients in the amount as given in Table 6 were mixed together. The blend so obtained was filled into capsules.

**TABLE 6**

| **Ingredient** | **Weight (mg/capsule)** |
|---|---|
| Omeprazole | 20.00 |
| Kollidon CL-M | 50.00 |
| Avicel PH 112 | 50.00 |
| Total | 120.00 |

The capsules were enteric coated in a Freund Hi-coater to a weight build-up of 10%.

### EXAMPLE 6

Omeprazole and Kollidon CL-M in amounts as given in Table 7 were mixed together. AKOMED R (fatty acid glyceride composed of caprylic / capric triglycerides and derived from coconut and/or palm kernel oils) and Gelucire 33/01 (a mixture of glycerides, e.g., mono-, di- and/or triglycerides of long chain fatty acids) were heated to 60°C for 20 minutes, stirred well and cooled to 30°C. The omeprazole and the Kollidon blend was granulated-with the liquid mix. The granules were screened through a No. 22 mesh sieve and filled into capsules. The capsules were enteric coated in a Freund Hi-coater to a weight build-up of 10% using the coating composition as given in Table 2.

**TABLE 7**

| **Ingredient** | **Weight (mg/capsule)** |
|---|---|
| Omeprazole | 20.00 |
| Cross-linked polyvinylpyrrolidone (Kollidon CL-M) | 100.00 |
| Gelucire 33/01 | 10.00 |
| AKOMED R | 20.00 |
| Total | 150.00 |

The enteric coated capsules of Examples 1 to 6 were tested as described under dissolution test (Method B) for delayed release (enteric coated) dosage forms in the United States Pharmacopoeia XXIII, page 1795. In the acid stage, omeprazole was not released from the capsules. The data for percent released in the buffer stage is given in Table 8.

**TABLE 8**

| **TIME (MINUTES)** | **MEAN CUMULATIVE PERCENT RELEASED** | | | | | |
|---|---|---|---|---|---|---|
| | **Ex. 1** | **Ex. 2** | **Ex. 3** | **Ex. 4** | **Ex. 5** | **Ex. 6** |
| 20 | 6.50 | 21.60 | 58.40 | 14.70 | 1.70 | 72.20 |
| 30 | 46.70 | 39.50 | 83.66 | 30.00 | 4.80 | 102.00 |
| 45 | 91.50 | 60.10 | 95.60 | 75.00 | 84.90 | 106.80 |

In another test, the enteric coated capsules of Examples I to 6 were kept in high density polyethylene bottles at 40°C/ 75% RH (Relative Humidity). The pharmaceutical compositions filled in the enteric capsules did not show any sign of instability such as change in color or appearance as given in Table 9.

**TABLE 9**

| | **OBSERVATION** | |
|---|---|---|
| **EXAMPLE No.** | **15 days, 40°C/75% RH** | **30 days, 40°C/75% RH** |
| 1 | No Change | No Change |
| 2 | No Change | No Change |
| 3 | No Change | No Change |
| 4 | No Change | No Change |
| 5 | No Change | No Change |
| 6 | No Change | No Change |

The Omeprazole content in the capsules stored as given above for a period of 30 days was determined by a stability-indicating HPLC method. The results are given in Table 10.

**TABLE 10**

| **EXAMPLE No.** | **Assay** |
|---|---|
| 1 | 105.10 |
| 2 | 100.19 |
| 3 | 99.85 |
| 4 | 99.66 |
| 5 | 98.07 |
| 6 | 95.20 |

While the invention has been described by reference to specific embodiments, this was for purposes of illustration only. Numerous alternative embodiments will be apparent to those skilled in the art and are considered to be within the scope of the claimed invention.

## Claims

1. A pharmaceutical composition which is stable and suitable for oral administration to a patient, comprising a mixture of omeprazole having gastric acid secretion inhibitory activity in an amount sufficient to inhibit gastric acid secretion in said patient, and a pharmaceutically acceptable carrier, said carrier comprising cross-linked polyvinylpyrrolidone, wherein said mixture is contained within a capsule shell and the capsule shell is either coated with an enteric material or is made from an enteric material **characterised in that** said carrier comprises about 10% to about 98% by weight of said mixture.

2. The composition of claim 1 wherein said carrier comprises about 50% to about 90% by weight of said mixture.

3. The composition of claim 1 wherein said mixture further comprises a fatty acid glyceride.

4. The composition of claim 1 wherein said mixture contained within said capsule shell is in the form of a powder blend.

5. The composition of claim 1 wherein said mixture contained within said capsule shell is in the form of granules.

6. A process for preparing a stable pharmaceutical composition which is suitable for oral administration, the composition comprising a mixture of omeprazole having gastric acid secretion inhibitory activity in an amount sufficient to inhibit gastric acid secretion in said patient, and about 10% to about 98% by weight of a pharmaceutically acceptable carrier, said carrier comprising cross-linked polyvinylpyrrolidone, wherein said mixture is contained within a capsule shell and the capsule shell is either coated with an enteric material or is made from an enteric material **characterised in that** said carrier comprises about 10% to about 98% by weight of said mixture, comprising mixing omeprazole having gastric acid secretion inhibitory activity in an amount sufficient to inhibit gastric acid secretion in a patient together with and a pharmaceutically acceptable carrier comprising crosslinked polyvinylpyrrolidone to form a mixture, and filling said mixture into a capsule shell which is either coated with an enteric material or is made from an enteric material.

7. The process of claim 6 further comprising mixing at least one pharmaceutically acceptable excipient into said mixture.

8. The process of claim 7 wherein said pharmaceutically acceptable excipient is a fatty acid glyceride.

9. The process of claim 6 comprising filling said mixture in the form of a powder blend into a capsule made from an enteric material.

10. The process of claim 6 comprising coating a capsule shell with an enteric material, and filling said mixture in the form of a powder blend into said capsule shell.

11. The process of claim 6 comprising granulating said mixture to produce granules, and filling said granules into a capsule shell made from an enteric material.

12. The process of claim 6 comprising granulating said mixture so as to form granules, coating a capsule shell with an enteric material, and filling said granules into said capsule shell.

## Patentansprüche

1. Pharmazeutische Zusammensetzung, welche stabil und für eine orale Verabreichung bei einem Patienten geeignet ist, enthaltend eine Mischung aus Omeprazol mit einer eine Magensäuresekretion inhibierenden Aktivität in einer Menge, die ausreichend ist, die Magensäuresekretion in dem Patienten zu inhibieren, und einem pharmazeutisch akzeptablen Träger, wobei der Träger vernetztes Polyvinylpyrrolidon enthält, wobei die Mischung innerhalb einer Kapselhülle enthalten ist und die Kapselhülle entweder mit einem darmlöslichen Material beschichtet ist oder aus einem darmlöslichen Material hergestellt ist, **dadurch gekennzeichnet, dass** der Träger ungefähr 10 Gew.-% bis ungefähr 98 Gew.-% der Mischung ausmacht.

2. Zusammensetzung nach Anspruch 1, wobei der Träger von ungefähr 50 Gew.-% bis ungefähr 90 Gew.-% in der Mischung enthalten ist.

3. Zusammensetzung nach Anspruch 1, wobei die Mischung des Weiteren ein Fettsäureglycerid enthält.

4. Zusammensetzung nach Anspruch 1, wobei die innerhalb der Kapselhülle enthaltende Mischung in Form einer Pulvermischung vorliegt.

5. Zusammensetzung nach Anspruch 1, wobei die in der Kapselhülle enthaltende Mischung in der Form von Granalien vorliegt.

6. Verfahren zur Herstellung einer stabilen pharmazeutischen Zusammensetzung, welche für eine orale Verabreichung geeignet ist, wobei die Zusammensetzung eine Mischung von Omeprazol mit einer eine Magensäuresekretion inhibierenden Aktivität in einer Menge, die zur Inhibierung der Magensäuresekretion in dem Patienten ausreichend ist, und ungefähr 10 Gew.-% bis ungefähr 98 Gew.-% eines pharmazeutisch akzeptablen Trägers, welcher vernetztes Polyvinylpyrrolidon aufweist, enthält, wobei die Mischung innerhalb einer Kapselhülle enthalten ist und die Kapselhülle entweder mit einem darmlöslichen Material beschichtet ist oder aus einem darmlöslichen Material hergestellt ist, **dadurch gekennzeichnet, dass** der Träger ungefähr 10 Gew.-% bis ungefähr 98 Gew.-% der Mischung ausmacht, umfassend das Mischen von eine Magensäuresekretion inhibierende Aktivität aufweisendem Omeprazol in einer Menge, die ausreichend ist, die Magensäuresekretion in einem Patienten zu inhibieren, zusammen mit einem pharmazeutisch akzeptablen Träger, welcher vernetztes Polyvinylpyrrolidon enthält, um eine Mischung zu ergeben, und Einfüllen der Mischung in eine Kapselhülle, welche entweder mit einem darmlöslichen Material beschichtet ist oder aus einem darmlöslichen Material hergestellt ist.

7. Verfahren nach Anspruch 6, des Weiteren umfassend die Mischung von wenigstens einem pharmazeutisch akzeptablen Arzneimittelträger in die Mischung.

8. Verfahren nach Anspruch 7, wobei der pharmazeutisch akzeptable Arzneimittelträger ein Fettsäureglycerid ist.

9. Verfahren nach Anspruch 6, wobei das Einfüllen der Mischung in Form einer Pulvermischung in eine aus einem darmlöslichen Material hergestellte Kapsel umfasst ist.

10. Verfahren nach Anspruch 6, wobei das Beschichten einer Kapselhülle mit einem darmlöslichen Material und Einfüllen der Mischung in Form einer Pulvermischung in die Kapselhülle umfasst ist.

11. Verfahren nach Anspruch 6 umfassend eine Granulierung der Mischung, um Granalien zu bilden, und Einfüllen der Granalien in eine aus einem darmlöslichen Material hergestellte Kapselhülle.

12. Verfahren nach Anspruch 6 umfassend eine Granulierung der Mischung, um so Granalien zu bilden, Beschichtung einer Kapselhülle mit einem darmlöslichen Material und Einfüllen der Granalien in die Kapselhülle.

## Revendications

1. Composition pharmaceutique qui est stable et appropriée pour une administration orale à un patient, comprenant un mélange d'oméprazole ayant une activité inhibitrice de sécrétion d'acide gastrique dans une quantité suffisante pour inhiber la sécrétion d'acide gastrique chez ledit patient, et un support pharmaceutiquement acceptable, ledit support comprenant de la polyvinylpyrrolidone réticulée, où ledit mélange est contenu à l'intérieur d'une enveloppe d'une gélule et l'enveloppe de la gélule est enrobée soit d'une matière gastrorésistante ou est réalisée à partir d'une matière gastrorésistante,
**caractérisée en ce que** ledit support comprend environ 10% à environ 98% en poids dudit mélange.

2. Composition de la revendication 1, dans laquelle ledit support comprend environ 50 % à environ 90 % en poids dudit mélange.

3. Composition de la revendication 1, dans laquelle ledit mélange comprend de plus un glycéride d'acide gras.

4. Composition de la revendication 1, dans laquelle ledit mélange contenu à l'intérieur de ladite enveloppe de gélule est sous la forme d'un mélange pulvérulent.

5. Composition de la revendication 1, dans laquelle ledit mélange contenu à l'intérieur de ladite enveloppe de gélule est sous la forme de granules.

6. Procédé pour préparer une composition pharmaceutique stable qui est appropriée pour une administration orale, la composition comprenant un mélange d'oméprazole ayant une activité inhibitrice de sécrétion d'acide gastrique dans une quantité suffisante pour inhiber la sécrétion d'acide gastrique chez ledit patient, et environ 10 % à environ 98 % en poids d'un support pharmaceutiquement acceptable, ledit support comprenant de la polyvinylpyrrolidone réticulée, où ledit mélange est contenu à l'intérieur d'une enveloppe de gélule et l'enveloppe de la gélule est enrobée soit d'une matière gastrorésistante ou est réalisée à partir d'une matière gastrorésistante,
**caractérisé en ce que** ledit support comprend environ 10 % à environ 98 % en poids dudit mélange, comprenant le mélange d'oméprazole ayant une activité inhibitrice de sécrétion d'acide gastrique dans une quantité suffisante pour inhiber la sécrétion d'acide gastrique chez un patient en même temps qu'un support pharmaceutiquement acceptable comprenant de la polyvinylpyrrolidone réticulée pour former un mélange, et le remplissage dudit mélange dans une enveloppe de gélule qui est enrobée soit avec une matière gastrorésistante ou est réalisée à partir d'une matière gastrorésistante.

7. Procédé de la revendication 6, consistant de plus à mélanger au moins un excipient pharmaceutiquement acceptable dans ledit mélange.

8. Procédé de la revendication 7, dans lequel ledit excipient pharmaceutiquement acceptable est un glycéride d'acide gras.

9. Procédé de la revendication 6, consistant à verser ledit mélange sous la forme d'un mélange pulvérulent dans une gélule réalisée en une matière gastrorésistante.

10. Procédé de la revendication 6, consistant à enrober une enveloppe de gélule avec une matière gastrorésistante, et à verser ledit mélange sous la forme d'un mélange pulvérulent dans ladite enveloppe de gélule.

11. Procédé de la revendication 6, consistant à mettre en granules ledit mélange pour produire des granules et à verser lesdits granules dans une enveloppe de gélule réalisée à partir d'une matière gastrorésistante.

12. Procédé de la revendication 6, consistant à mettre en granules ledit mélange de façon à former des granules, à enrober une enveloppe de gélule avec une matière gastrorésistante et à verser lesdits granules dans ladite enveloppe de gélule.
